Europäisches Patentamt

European Patent Office

Office européen des brevets

(11) Publication number: **0 361 720**
**A1**

## (12) EUROPEAN PATENT APPLICATION

(21) Application number: **89309170.2**

(51) Int. Cl.⁵: **A61K 37/14**

(22) Date of filing: **08.09.89**

(30) Priority: **08.09.88 IL 87708**

(43) Date of publication of application:
**04.04.90 Bulletin 90/14**

(84) Designated Contracting States:
**AT DE ES FR GB IT SE**

(71) Applicant: **Technion Research & Development Foundation Ltd.**
**Technion City**
**IL-32000 Haifa(IL)**

(72) Inventor: **Ilan, Ehud**
**14 Mandelstam Street**
**Tel-Aviv(IL)**
Inventor: **Lotan, Noah**
**27 Shimkin Street**
**Haifa(IL)**
Inventor: **Cohen, Tova**
**36 Nesher Street**
**Carmiel(IL)**
Inventor: **Sideman, Samuel**
**13 Hachsharat Hayeshuv Street**
**Haifa(IL)**

(74) Representative: **Clifford, Frederick Alan et al**
**MARKS & CLERK 57/60 Lincoln's Inn Fields**
**London WC2A 3LS(GB)**

(54) **Hemoglobin-based blood substitute and method for the preparation thereof.**

(57) Hemoglobin-based blood substitute resulted from stroma-free hemoglobin, intramolecularly stabilized, modified with pyridoxal-5'-phosphate and subsequently polymerized under anaerobic conditions at an extent of above 40%. The new blood substitute is not toxic and possesses the proper oxygen affinity, adequate oncotic pressure and percentage of the undesirable methemoglobin, substantially equal to those of whole human blood. Among the main characteristic properties of the novel hemoglobin-based blood substitute the following may be mentioned: appropriate lifetime in circulation upon reconstitution in an appropriate solvent, good oxygen binding and delivery characteristics and appropriate oxygen transport capacity. The method for its preparation is simple and requires common reagents.

EP 0 361 720 A1

The present invention relates to blood substitute material. More particularly, the invention relates to a hemoglobin-based blood substitute material and to a method for the preparation thereof.

## BACKGROUND OF THE INVENTION

As known, blood transfusion has become a universally accepted, life-saving procedure used in modern clinical medicine. Blood losses at substantial amounts are very dangerous, since they decrease the blood volume and pressure, limit the oxygen supply to vital tissues and slow the metabolic processes. However, the collection of blood for transfusion, its storage and administration is a complex subject which involves serious problems such as limited resources, compatibility and risk of transmitting certain diseases (e.g. hepatitis, AIDS, malaria, etc). Moreover, when considering performing the blood transfusion in the field, even more complicated problems are encountered, not only of medical origin but also logistic, such as availability at site, transport and storage conditions, etc.

In view of the above problems, the search for blood substitutes was seriously considered and a number of related papers and patents can be found in the respective literature.

Two main groups of blood substitutes are mentioned in the literature:(a) perfluorocarbons-based,and (b) hemoglobin-based. Perfluorocarbons-based substitutes are cyclic -, branched, or straight-chain molecules, in which hydrogen atoms have been replaced with fluorine. Due to the strength of the carbon-fluorine bond, these compounds are generally considered as very inert, both chemically and biologically. They are capable of absorbing oxygen by physical dissolution, the amount absorbed being linearly dependent on the composition of the equilibrating gas phase. However, recent studies have shown that perfluorocarbons have several disadvantages such as adverse reactions on the immune system, hematological disturbances, alterations in prostaglandin metabolism, impairment of the coagulation process, etc.

The present invention relates to a hemoglobin-based substitute, and therefore some more discussion will be presented on this subject. As known, hemoglobin binds oxygen in a process having the characteristic of non-linearity, and a chemical species known as oxyhemoglobin is thus formed. An ideal hemoglobin-based blood substitute should have the ability to transport and supply oxygen from the lungs to vital tissues and organs, and to transport carbon dioxide from these tissues and organs back to the lungs. In the same time, it must have minimal side effects, so that could be tolerated in large doses.

It is also known that hemoglobin is a tetrameric protein, composed of equal numbers of two different protein chains, alpha and beta. Under physiological conditions, the predominant molecular species is the said tetramer, having a molecular weight of about 65,000. In mammals, hemoglobin is present in erythrocytes, the membrane of which contains stroma which consists of phospholipids, cholesterol, and proteins.

In the followings, the terms stroma-free hemoglobin and stroma depleted hemoglobin can be used interchangeably, and both mean a product from which essentially all the stromal components were removed.

Attempts have been made to use stroma-free hemoglobin solutions as blood substitute,exhibiting oxygen transport capabilities. These solutions endure storage at room temperature for limited periods of time, without changes in their properties. However, oxygen binds to hemoglobin very strongly, so that only a small part of the bound oxygen is released to the cells at the oxygen tension normally prevailing in tissues. Also, not all of the important characteristics of these solutions are equal to those of human blood. Thus for instance, at the required concentration, the corresponding oncotic pressure is far from that of normal blood. Further investigations have also shown that stroma is a toxic factor, probably by causing thrombosis of the small renal vasculature.

In view of some favorable properties possessed by stroma-free hemoglobin, intensive work was done to improve the disadvantages encountered with its use as blood substitute. One approach was to modify its structure in order to decrease its oxygen affinity. For this purpose, it was suggested by Benesch et al [Biochemistry 11, 3576-3582 (1972)] to treat the stroma-free hemoglobin with pyridoxal-5′-phosphate (hereafter referred to as PLP). The product obtained, pyridoxal-5′-phosphate hemoglobin (hereafter referred to as PLP-hemoglobin), indeed has a satisfactory oxygen affinity, but the intravascular life time is too short compared to what is required for a resuscitation material.

Based on the same approach of modifying the structure of stroma-free hemoglobin, it is described in the U.S. Patent Number 4,061,736 a method for obtaining an intramolecularly crosslinked stroma-free hemoglobin, by reacting the latter with a reagent selected from aldehydes, dialdehydes, activated ureas, derivatives of carboxylic acids, heterocyclic triazines and halogenated aromatic cycloalkanes.

According to U.S. Patent No. 4,598,064, stroma-free hemoglobin is modified by intramolecular crosslinking between the alpha chains of the tetramer, using an acylating agent. It is claimed that the product obtained is an effective blood substitute having suitable oxygen binding characteristics, such as low oxygen affinity, being particularly useful in the treatment of ischemia. Another approach, which was also investigated, consists in the modification of hemoglobin in an attempt to improve both the oxygen binding characteristics and the intravascular retention. Thus according to PCT Patent Application No. 84/04248, hemoglobin is first covalently crosslinked intramolecularly between the two beta chains, and then reacted with PLP. It is claimed that the product thus obtained is a stable, oxygen carrying material, capable of delivering oxygen to perfused tissue, and advantageously remaining in the intravascular space. However, one of the main disadvantages of the blood substitute obtained according to the method described therein is its limited oxygen carrying capacity.

Still another approach involves the polymerization of PLP-hemoglobin [G.S. Moss et al, Surgery 95, 249-255 (1984)]. However, the product thus obtained has, again, a limited oxygen carrying capacity.

Summing-up the above short review of the prior art, it should be concluded that there are, indeed, some improvements towards the use of hemoglobin-based blood substitutes beyond the use of stroma-free hemoglobin. Blood substitutes containing one or more characteristics of normal blood can thus be encountered, but still there is a long felt need for a blood substitute which will possess most of the characteristics of the normal blood. It is an object of the present invention to provide a blood substitute which possesses most of the characteristics of the normal blood. It is another object of the present invention to provide a hemoglobin-based blood substitute which possesses most of the characteristics of the normal blood. It is yet another object of the present invention to provide a hemoglobin-based blood substitute to be useful for clinical use. A further object of the present invention is to provide a simple method for obtaining a hemoglobin-based blood substitute useful for clinical use.

## BRIEF DESCRIPTION OF THE INVENTION

The invention relates to a hemoglobin-based blood substitute, obtained from stroma-free hemoglobin by the following sequential procedure:

(a) intramolecular stabilization of the stroma-free hemoglobin;

(b) pyridoxylation of the intramolecularly stabilized stroma-free hemoglobin; and

(c) polymerization at an extent of above 40%, under anaerobic conditions, of the pyridoxylated intramolecularly stabilized stroma-free hemoglobin.

In the followings, the material obtained from step (a) above will be referred to as TS-hemoglobin (intramolecularly-stabilized hemoglobin); the material obtained from step (b) above will be referred to as PLP-TS-hemoglobin (pyridoxylated-intramolecularly-stabilized hemoglobin) and the material obtained from step (c) above will be referred to as Poly-PLP-TS-hemoglobin (polymerized-pyridoxylated-intramolecularly stabilized hemoglobin).

The final product obtained, when reconstituted with an appropriate solvent, yields a blood substitute solution which is characterized by properties which are substantially equal to those of whole human blood and which are highly improved when compared to those solutions of unmodified hemoglobin. Table 1 below summarizes the main properties of whole human blood under normal physiological conditions, of a solution of unmodified hemoglobin [the values for unmodified hemoglobin solution were taken from Lakshman et al , Journal of Surgical Research 30, 14-20 (1981)], and of a solution of the hemoglobin-based blood substitute obtained according to the present invention.

TABLE 1.

| Main properties of whole human blood, of a solution of unmodified hemoglobin and of a solution of the blood substitute material prepared according to the present invention | | | |
|---|---|---|---|
| Property | Whole human blood | Solution of Unmodified Hemoglobin | Solution of Blood Substitute |
| -Hemoglobin conc. (g/100 ml) | 12-18 | 7- 8 | 10-18 |
| -$P_{50}$ (torr) | 26-29 | 12-14 | 25-28 |
| -COP (torr) | 25-29 | 20-25 | 24-31 |
| -Methemoglobin content (% of total Hb) | up to 5 | 3- 8 | 2.5-5 |
| $P_{50}$ = the partial pressure of oxygen at which 50% of hemoglobin sites are saturated with oxygen; measured at normal physiological conditions: pH = 7.4, $pCO_2$ = 40 torr, temperature = 37.0°C. COP = colloid osmotic (i.e. oncotic) pressure. | | | |

As appears from the above Table 1, a solution of the blood substitute material prepared according to the present invention is characterized by its proper oxygen affinity, as well as by its adequate COP at normal physiological concentrations of hemoglobin.

The blood substitute material prepared according to the present invention is characterized by the following advantageous properties:

a. Is not toxic.

b. Possesses appropriate lifetime in circulation.

c. The solution obtained upon reconstitution in an appropriate solvent possesses good oxygen binding and delivery characteristics.

d. The solution obtained upon reconstitution in an appropriate solvent possesses normal oncotic pressure at physiological concentrations of hemoglobin, and correspondingly appropriate oxygen transport capacity.

In view of the above, transfusion with this blood substitute solution is much more convenient to perform than transfusion with whole blood, because there is no need to "type and crossmatch" prior to transfusion and thus lives may be saved by saving precious time.

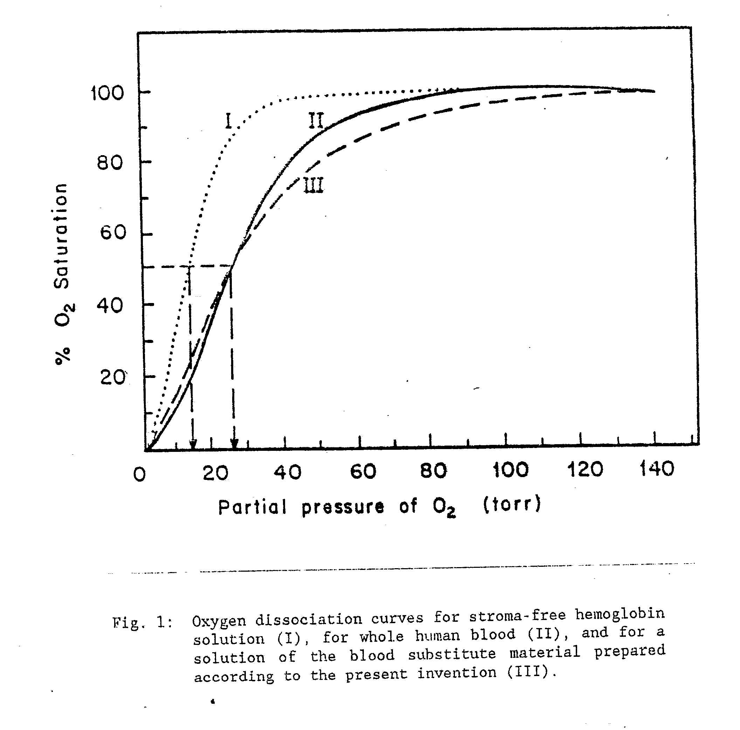

· One of the important properties required for a blood substitute is to have an appropriate oxygen dissociation curve. In Figure 1 there is a diagram illustrating in a clear manner this property for whole human blood (II), for the solution of the blood substitute prepared according to the present invention (III), and for the solution of stroma-free hemoglobin (I), which is the starting material for said blood substitute material. As appears from Figure 1, the $P_{50}$ of the blood substitute solution (25-28 torr) is virtually equal to that of whole human blood (26-29 torr) whereas the $P_{50}$ of stroma-free hemoglobin solution (12-14 torr) is very much lower than that of whole blood.

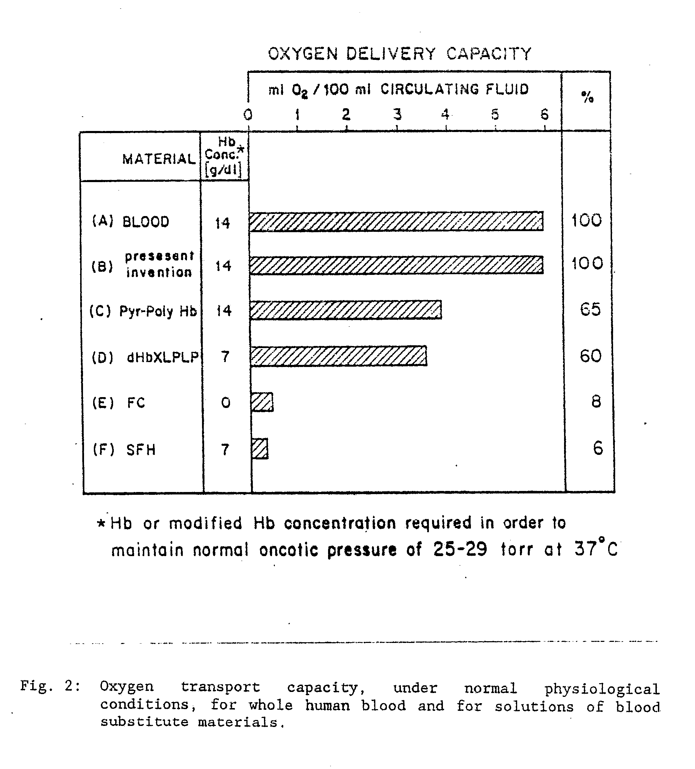

Another important property required for a blood substitute is to have the appropriate capacity for carrying oxygen from the lungs to the tissues. This characteristic is presented schematically in Figure 2, for whole human blood (A), for a solution of the blood-substitute prepared according to the present invention (B), for the blood-substitute solutions described in the prior art and obtained from polymerized PLP-hemoglobin (C) [G.S. Moss et al., Surgery 95, 249-255 (1984)] and from intramolecularly crosslinked pyridoxylated hemoglobin (D) (PCT Patent Application No. 84/04248), for Fluosol DA fluorocarbon emulsion (E) [H. Ohyanagi and Y. Saitoh, Int. J. Art. Organs 9, 363-368 (1986)] and for stroma-free hemoglobin solution exhibing the required oncotic pressure (F). As appears from this Figure, the oxygen carrying capacity - measured relatively to whole human blood at normal physiological conditions - is 100% for the blood substitute prepared according to the present invention, compared with 65% or 60% for the prior art blood-substitute solutions, 8% for fluorocarbons, and 6% for the stroma-free hemoglobin solution.

The hemoglobin - based blood substitute material prepared according to the present invention is much superior to the perfluoro-chemicals suggested as blood substitutes in casualties suffering from hypovolemic shocks, due to its ability to carry appropriate amounts of oxygen when the victim is ventilated with room air. It may alos be used to treat acute myocardial infarcts, and cerebral vascular accidents.

4

The improved properties of the blood-substitute prepared according to the present invention result from the polymerization of the PLP-TS-hemoglobin. It was found that this polymerization should be carried out only in absence of oxygen, and under conditions in which the extent of polymerization achieved is above 40%. Solutions of blood substitutes with physiological hemoglobin concentrations of 12-18% will necessarily possess inadequately high oncotic pressure.

It was found according to the present invention that the most preferred extent of polymerization is in the range of between 50% to 80%.

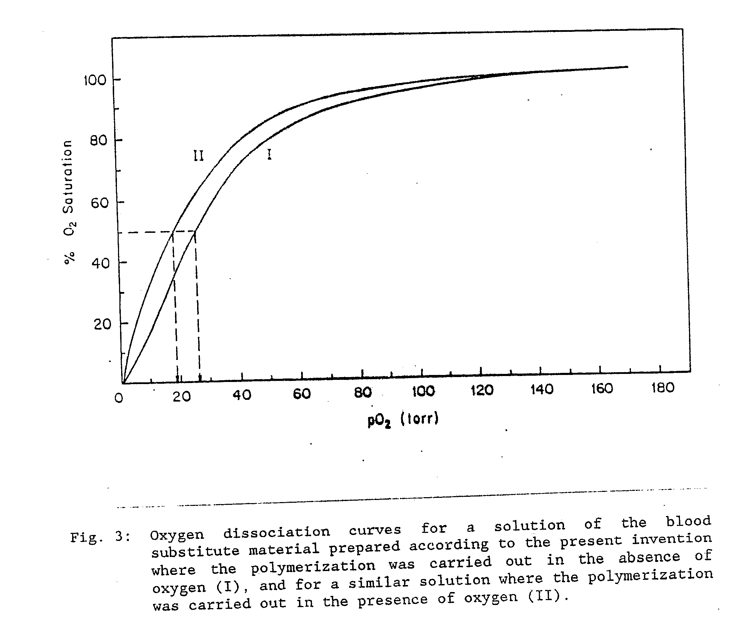

In Figure 3 are presented two graphs which correlate the percentage of oxygen saturation and the partial pressure of oxygen (expressed in torr). Graph I is the oxygen dissociation curve of a solution of the blood substitute prepared according to the present invention, particularly in case that the said polymerization is carried out in the absence of oxygen, whereas graph II when said polymerization is carried out in the presence of oxygen. In the following Table 2 the main characteristic properties of the products obtained in the absence and presence of oxygen are presented.

TABLE 2.

| Characteristic properties of solution of the blood substitute material prepared according to the present invention when the polymerization is carried out in the presence or absence of oxygen | | |
|---|---|---|
| Property | Product obtained in the absence of oxygen | Product obtained in the presence of oxygen |
| - Hemoglobin concentration (g/100 ml) | 12.2 | 14.2 |
| - Methemoglobin content (% of total hemoglobin) | 2.7 | 5.6 |
| - $P_{50}$ (at pH = 7.4; $pCO_2$ = 40 torr and T = 37°C) | 26 | 19 |

From the above data it clearly appears that the polymerization in the presence of oxygen leads to a product with a $P_{50}$ of 19 toor, which is significantly lower than that of the corresponding product obtained by polymerization in the absence of oxygen ($P_{50}$ = 26 torr). Also, the methemoglobin content in the product is much higher when the polymerization is carried out in the presence of oxygen rather than in its absence (5.6% and 2.7%, respectively) which is a clear disadvantage.

Polymerization reagents for this step should be selected from various compounds such as glutaraldehyde or succinic dialdehyde or precursors thereof such as 2-ethoxy-3,4-dihydro-1,2-pyran, 2,5-diethoxy tetrahydrofuran, 2-amino-4,6-dichloro-S-triazine, bis-diazobenzidine 2,2-sulfonic acid, 1,5-difluoro-2,4-dinitrobenzene, ethylene glycol diglycidyl ether, butadiene diepoxide etc.

Generally, the polymerization reagent is added to a buffered solution of PLP-TS-hemoglobin having a pH between 6.5 and 8.5, and preferably of 7.5.

According to a most preferred embodiment, the polymerized product obtained is quenched in an amine solution in order to avoid that moieties containing active groups of the polymerization reagent remain in the final product. Accordingly, the amine should be added in an amount sufficient to quench all the unreacted groups of the polymerization reagent. Although amines such as ethanolamine and lysine are preferred reagents, other reagents may be utilized,such as bisulfites, alcohols, isocyanates and mercaptans.

An additional optional step which, if carried out anaerobically, would avoid an increase in the methemoglobin content, is reduction with sodium borohydride. This step ensures the saturation of the double bonds formed by the polymerization reagent, thus giving extra stability to the polymerized hemoglobin. Since, at the introduction of sodium borohydride, apart from the reduction of the double bonds, the residual carbonyl groups introduced by the polymerization reagent are also reduced, the addition of a quenching agent is generally superfluous. In most cases, only one additional step after the polymerization, i.e. quenching or reduction with borohydride will be indicated, but for specific cases such as when extra stability of the polymerized product is needed together with precise regulation of the polymerization step, the two additional steps might be required. A person skilled in the art, will select the proper step according to the specific requirement for the blood-substitute.

One of the advantages of the process for obtaining the hemoglobin-based substitute according to the present invention, is the use of common reagents, commercially available or easily synthesized, which are generally utilized for this purpose. The product obtained may be lyophilized, in the presence of 3% glucose or other protective agents as known in the art, without changing its oxygen-transporting properties as a potential resuscittion fluid upon reconstitution.

5

In summary,the blood-substitute hemoglobin-based material prepared according to the present invention is a superior product for the rapid treatment of hypovolemic shock, because it can be used by paramedical personnel without delay. It is easy to prepare; has a long storage life and delivers oxygen to perfused tissues when the victim is inhaling room air. It may be used in many critical situations other than combat casualty and hypovolemic shock. It should be an ideal substitute of whole blood for use as priming fluid in extracorooreal pumps employed in cardiac surgery, or in extracorporeal devices employed in blood treatments such as hemoperfusion and hemodialysis. It also could be used to treat ischemic episodes including sludging and pain in sickle cell crises. It could be used as an organ perfusant to maintain organ viability until transplanted.

## DETAILED DESCRIPTION OF THE INVENTION

The first step requires the obtaining of stroma-free hemoglobin. This is carried out starting with packed red blood cells or whole blood from various mammalian species. There are several known methods for obtaining the stroma-free hemoglobin, most of them producing a suitable starting material for the present invention. As known, when stroma is present in hemoglobin preparations in large amounts, it interferes with renal function. Investigations in the past had shown that stroma caused thrombosis of the small renal vasculature. It was already described in many references that, by removal of most of the stroma from hemoglobin solution, this disadvantage is eliminated.

The method selected to be used in the present invention for preparing the stroma-free hemoglobin is fully described in a paper by De Venuto et al. [J. Lab. Clin. Med. 89, 509-516, (1977)].

The second step involves the intramolecular stabilization of the stroma-free hemoglobin. This is carried out by a chemical reaction with a compound selected from haloesters of dicarboxylic acids, such as bis(3,5-dibromosalicyl)glutarate; bis (3,5-dibromosalicyl)adipate, bis(3,5-dibromosalicyl)fumarate; bis(3,5-dichlorosalicyl)fumarate; bis(3,5-diiodosalicyl)fumarate ;bis(3,5-dibromosalicyl)succinate. The reaction introduces a covalently bound glutarate (or adipate, succinate, fumarate etc.) bridge between the two beta chains of the hemoglobin molecule. This modification brings about an improved intravascular retention and oxygen transport capability of the product compared to the original stroma-free hemoglobin solution. The $P_{50}$, at normal physiological conditions, of the solution after this intramolecular stabilization increases to 23 torr, a value approaching the normal $P_{50}$ of whole human blood.There are several variations for carrying out this reaction, but in particular suitable for the present invention was the route described by R. W. Tye et al. (Advances in Blood Substitute Research, R. B. Bolin, R. P. Geyer and G. J. Nemo eds., Alan R. Liss Inc. 1983, p. 41-49).

In the third step, the TS-hemoglobin is pyridoxylated using a method similar to the one described by Benesch et al. [Biochemistry 11, 3576-3582, (1972)]. As mentioned in the literature, the resulting solution of PLP-TS-hemoglobin (see R.W. Tye et al. Advances in Blood Substitute Research, R.B.Bolin, R.P.Geyer and G.J.Nemo eds., Alan R. Liss Inc. 1983, p. 41-49) exhibits a $P_{50}$ of about 28 torr, at normal physiological conditions, compared with 12-14 torr shown by a corresponding solution of unmodified hemoglobin.

The fourth step is the crucial one for obtaining the blood substitute with the improved life time in circulation and adequate oncotic pressure. This step involves the polymerization under anaerobic conditions of the PLP-TS-hemoglobin obtained in the previous step at an extent of above 40% and preferably in the range of between 50% to 80%. This polymerization is carried out using polyfunctional agents having groups that are capable of reacting with amino groups and other linkable sites on the hemoglobin molecule. In particular useful is the reaction with bi- or polyfunctional carbonyl containing compounds. Within this group of materials, glutaraldehyde was found to produce excellent results. It was found that the preferred molar ratio of polymerizing reagent - to - hemoglobin ranges between 1:1 to 30:1. The oxygen-free environment, required in this step is maintained by continuously bubbling a stream of inert gas such as nitrogen. Preferably, the reaction is carried out on a PLP-TS-hemoglobin solution having a hemoglobin concentration of between 7 to 16% w/v. In order to prevent foaming during the reaction, addition of an antifoaming reagent is suggested.

Optionally, at the end of this step, the product may be quenched and/or reduced with sodium borohydride in order to improve its qualities (see also: Brief Description of the Invention). The obtained product possesses the properties desired for a blood-substitute.

## EXAMPLE.

**STEP 1.**

A solution of washed crystals of stroma-free hemoglobin was prepared as per the method of De Venuto et al.[J.Lab. Clin. Med. 89, 509-516 (1977)] and was dialysed at 4°C, first twice against distilled water (1:24 by volume) in order to reduce the potassium and phosphate content and then three times more (1:16 by volume) against Tris-HCl buffer (0.1 M, pH 7.4, also containing 0.2 g/l ascorbic acid and 1 g/l glucose). At the end of this procedure, the pH of the retenate solution was 7.4.

In order to ensure sterility to the resulted solution, sodium penicillin G, gentamycin and streptomycin sulfate were added to final concentrations of:
- 50,000 units/l sodium penicillin G.
- 50 mg/l gentamycin; and
- 50 mg/l streptomycin sulfate.

**STEP 2**

An amount of 50 ml of the stroma-free hemoglobin solution obtained in Step 1, with a concentration of 15-20% w/v was introduced into a 250 ml closed Pyrex container together with 0.25 ml of caprylic alcohol (as antifoaming agent). An oxygen-free environment was achieved by bubbling continuously a stream of nitrogen, and a deoxy genated solution was added, consisting of bis-(3,5-dibromosalicyl)fumarate (2 moles/mole of hemoglobin) dissolved in 5.0 ml Tris-HCl buffer (0.5 M, pH 8.0). The reaction was carried out in the absence of oxygen, a stream of nitrogen gas being bubbled continuously for about two hours, at 35°C.

**STEP 3.**

In this step, there is a pyridoxylation of the TS-hemoglobin product obtained in Step 2. In the same container, a deoxygenated solution was added, consisting of PLP (10 moles/mole of hemoglobin) dissolved in a mixture of 5.0 ml Tris-HCl buffer (1.0 M, pH 7.4) and 2.5 ml Tris-HCl buffer (1.0 M, pH 8.0). The reaction between TS-hemoglobin and PLP was carried out under continuous nitrogen bubbling for about 1 hour at about 25°C. The resulting solution was then treated with 1.5 ml of deoxygenated sodium borohydride solution (20 moles/mole of hemoglobin) in 1 mM NaOH. This reaction was carried out for two hours at 25°C under nitrogen bubbling. The resulting mixture was twice centrifuged, each time for 30 minutes at 4°C and 12,000 X g, in order to eliminate particular matter. The clear solution thus obtained was subsequently dialyzed four times at 4°C each time against 20 volumes of sodium phosphate buffer (0.05 M, pH 7.5) also contain ing 0.2 g/l ascorbic acid. At the end of this procedure, the reagents for ensuring sterility were added to the retenate to reach the concentrations as mentioned in Step 1 for final stroma- free hemoglobin solution.

**STEP 4.**

An amount of 30 ml solution of the PLP-TS-hemoglobin, obtained in Step 3, and containing 15% w/v hemoglobin, was introduced into a 100 ml closed Pyrex container together with 0.15 ml of caprylic alcohol (as antifoaming agent). A continuous stream of nitrogen gas was bubbled through the solution for about 1.5 hours. To the resulted deoxygenated solution, 1.4 ml of deoxygenated solution of glutaraldehyde (2.5% w/v), in sodium phosphate buffer (0.05 M, pH 7.5), was slowly added, while the steam of nitrogen gas continued. The reaction was continued for about three hours at 25°C, still under a stream of nitrogen gas. The extent of polymerization was found to be 58%.

In order to ensure the elimination of the residual active groups, the product obtained above was quenched with 3.0 ml deoxygenated solution of 1.0 M ethanolamine in sodium phosphate buffer (0.05 M), the pH of which was adjusted to 7.5 with phosphoric acid. After 1 hour quenching at 25°C, 0.9 ml of sodium borohydride solution (20 moles/mole of hemoglobin) in 1 mM NaOH were added anaerobically, and nitrogen gas bubbling into the solution was continued. After 2 hours, the reaction was terminated by dialysis, at 4°C, four times (1:20 v/v each time) against a standard kidney dialysis fluid [for composition see: De Venuto et al., J. Lab. Clin. Med. 89, 509-516 (1977)] also containing 0.2 g/l ascorbic acid, the pH being adjusted to 7.4 with lactic acid. The hemoglobin-based blood substitute solution was further treated (sterilization etc.) to obtain the transfusion solution as known in the art.

Preliminary toxicity tests were performed on seven white mice with transfusion solution of the blood substitute material obtained according to the present invention. Thus, an amount of 0.1 ml of the blood substitute solution containing 12.2 g/100 ml hemoglobin was injected through the tail of each mouse. Normal behaviour of the mice was noticed until they were sacrificed fifteen days after the transfusion.

Although in the specification the utilization of human hemoglobin was illustrated, it should be understood that other hemoglobin sources, such as bovine or porcine may be conceived to be utilized.

## Claims

1. Hemoglobin-based blood substitute, resulted from stroma-free hemoglobin, intramolecularly stabilized, pyridoxylated, and subsequently polymerized under anaerobic conditions at an extent of above 40%.

2. Hemoglobin-based blood substitute material according to Claim 1, possessing a hemoglobin concentration in the range of 10 to 18 g/100 ml.

3. Hemoglobin-based blood substitute material according to Claims 1 and 2, possessing a $P_{50}$ in the range of 25 to 28 torr.

4. Hemoglobin-based blood substitute material according to Claims 1 to 3, possessing a colloid osmotic pressure in the range of 24 to 31 torr.

5. Hemoglobin-based blood substitute material according to Claims 1 to 4, possessing a methemoglobin content in the range 2.5% to 5% of the total hemoglobin.

6. Hemoglobin-based blood substitute according to Claims 1 to 5, wherein the hemoglobin is derived from whole human blood, or packed human red blood cells.

7. Hemoglobin-based blood substitute according to Claims 1 to 6, wherein the extent of the polymerization is above 50%.

8. Hemoglobin-based blood substitute according to Claim 7, wherein the extent of polymerization is in the range of between 50% to 80%.

9. A method for obtaining hemoglobin-based blood substitute which comprises the steps of:

(a) producing stroma-free hemoglobin;

(b) intramolecular stabilization of the stroma-free hemoglobin by a reaction with a halo-ester of dicarboxylic acid;

(c) pyridoxylation of the TS-hemoglobin; and

(d) polymerization at an extent of about 40% under anaerobic conditions of the PLP-TS-hemoglobin using a polyfunctional agent having groups capable of reacting with linkable sites on the hemoglobin molecule.

10. A method according to Claim 9, wherein the polyfunctional agent used in step (d) contains a carbonyl group.

11. A method according to Claim 10, wherein said polyfunctional group is glutaraldehyde or precursors thereof.

12. A method according to Claims 10 or 11, wherein the molar ratio between the polymerizing reagent and hemoglobin is between 1:1 to 30:1.

13. A method according to Claims 9 to 12, wherein the PLP-TS-hemoglobin solution used in the polymerization has a hemoglobin concentration in the range of between 7% to 16% (w/v).

14. A method according to Claims 9 to 13, wherein the Poly-PLP-TS-hemoglobin is quenched in a quencher solution.

15. A method according to Claim 14, wherein said quencher is selected from amines, bisulfites, alcohols, isocyanates and mercaptans.

16. A method according to Claim 15, wherein said amine is selected from the group consisting of ethanolamine and lysine.

17. A method according to Claims 9 to 16, wherein the Poly-PLP-TS-hemoglobin is reduced by a solution of sodium borohydride.

18. Hemoglobin-based blood substitute substantially as described in the specification and claimed in any one of the Claims 1 to 7.

19. A method for obtaining hemoglobin-based blood substitute substantially as described in the specification and claimed in any one of Claims 9 to 17.

8

Fig. 1: Oxygen dissociation curves for stroma-free hemoglobin solution (I), for whole human blood (II), and for a solution of the blood substitute material prepared according to the present invention (III).

## OXYGEN DELIVERY CAPACITY

| MATERIAL | Hb Conc.* [g/dl] | ml $O_2$ / 100 ml CIRCULATING FLUID | % |
|---|---|---|---|
| (A) BLOOD | 14 | | 100 |
| (B) present invention | 14 | | 100 |
| (C) Pyr-Poly Hb | 14 | | 65 |
| (D) dHbXLPLP | 7 | | 60 |
| (E) FC | 0 | | 8 |
| (F) SFH | 7 | | 6 |

*Hb or modified Hb concentration required in order to maintain normal oncotic pressure of 25-29 torr at 37°C

Fig. 2: Oxygen transport capacity, under normal physiological conditions, for whole human blood and for solutions of blood substitute materials.

Fig. 3: Oxygen dissociation curves for a solution of the blood substitute material prepared according to the present invention where the polymerization was carried out in the absence of oxygen (I), and for a similar solution where the polymerization was carried out in the presence of oxygen (II).

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | A.S.A.I.O. TRANSACTIONS (AMERICAN SOCIETY FOR ARTIFICIAL INTERNAL ORGANS), vol. 33, no. 4, December 1987, pages 819-823, Hagerstown, MD, US; T.M.S. CHANG et al.: "Blood substitutes based on modified hemoglobin and fluorochemicals" * Page 829, right-hand column, lines 3-20 * | 1-19 | A 61 K 37/14 |
| Y | WO-A-8 707 832 (NORTHFIELD LABORATORIES) * Page 14, line 23 - page 20, line 20 * | 1-19 | |
| A | WO-A-8 803 408 (BIOPURE CORP.) | | |
| A | US-A-4 584 130 (BUCCI) | | |
| A | INT. J. ARTRITIC ORGANS, vol. 6, no. 6, 1983, pages 319-320; R. STABILINI et al.: "A pyridoxylated polymerized hemoglobin solution as oxygen carrying substitute" | | TECHNICAL FIELDS SEARCHED (Int. Cl.5) |
| A | APPL. BIOCHEM. BIOTECHNOL., vol. 10, 1984, pages 133-141, The Humana Press Inc.; P.E. KEIPERT et al.: "Preparation and in vitro characteristics of a blood substitute based on pyridoxylated polyhemoglobin" | | A 61 K |
| A | TRANSFUSION, vol. 23, no. 2, 1983, pages 158-162, J.B. LIPPINCOTT CO.; L.R. SEHGAI et al.: "Preparation and in vitro characteristics of polymerized pyridoxylated hemoglobin" | | |

The present search report has been drawn up for all claims

| Place of search | Date of completion of the search | Examiner |
|---|---|---|
| THE HAGUE | 08-12-1989 | TURMO Y BLANCO C.E. |

EPO FORM 1503 03.82 (P0401)